# EUROPEAN PATENT APPLICATION

(11) **EP 4 847 554 A1**
(43) Date of publication of application: **02.12.2026**
(21) Application number: 25776507.3
(22) Date of filing: 19.03.2025
(51) Int. Cl.: C08J 3/07, A61F 13/15, A61F 13/53, C08F 8/44, C08J 3/16

(54) **METHOD FOR PRODUCING POLYMER DISPERSION AND METHOD FOR PRODUCING WATER-ABSORBING RESIN PARTICLE**

(30) Priority: 28.03.2024 JP 2024055017
(71) Applicant: Sumitomo Seika Chemicals Co., Ltd., Kako-gun, Hyogo 675-0145 (JP)
(72) Inventor: SAKAI, Yuka, Himeji-shi, Hyogo 672-8076 (JP)
(74) Representative: Hasegawa, Kan
(86) International application number: PCT/JP2025/010693
(87) International publication number: WO 2025/205320

(57) **Abstract**

A production method for a polymer dispersion liquid includes a step of preparing a polymer solution containing a polymer in which a crosslinked structure of a water-absorbent resin has been cleaved, and a step of bringing a poor solvent for the polymer into contact with the polymer solution to precipitate a solid polymer, thereby obtaining a polymer dispersion liquid in which the solid polymer is dispersed, wherein a transmittance of the polymer dispersion liquid at a wavelength of 425 nm and an optical path length of 1 cm is 0.85% or less.

## Description

### Technical Field

The present invention relates to a method for producing a polymer dispersion liquid and a method for producing water-absorbent resin particles.

### Background Art

Generally, disposable sanitary products are configured to contain pulp fibers and a water-absorbent resin between a water-impermeable cover sheet and a water-permeable non-woven fabric, and the water-absorbent resin swells by containing moisture so that excrement can be absorbed. After such sanitary products are used, the sanitary products are treated by incineration or landfilling, but in recent years, the collection and reuse of components from used sanitary products have been examined. For example, Patent Literature 1 discloses a technique for decomposing a water-absorbent resin with a focus on cleavage of a crosslinked portion in the water-absorbent resin, and a technique for collecting a water-soluble recycled polymer obtained by decomposing a water-absorbent resin as an aggregate by crosslinking with a multivalent metal ion.

### Citation List

### Patent Literature

[Patent Literature 1] Japanese Unexamined Patent Publication No. 2020-49398

### Summary of Invention

### Technical Problem

When a polymer solution containing a polymer in which a crosslinked structure of a water-absorbent resin has been cleaved is re-crosslinked by a reverse-phase suspension system to regenerate a water-absorbent resin, if the polymer exhibits an ideal shape (true spherical shape) in a dispersion medium, the water-absorbent resin regenerated from the polymer also tends to be true spherical. According to the findings of the present inventors, when an attempt is made to regenerate a water-absorbent resin by subjecting the polymer solution as-is to re-crosslinking in a reverse-phase suspension system, it is difficult for the polymer to become true spherical in the dispersion medium, and therefore it is difficult to regenerate a true spherical water-absorbent resin.

Accordingly, one aspect of the present invention relates to a method for producing a polymer dispersion liquid in which a polymer tends to become true spherical in a dispersion medium in a reverse-phase suspension system.

### Solution to Problem

One aspect of the present disclosure includes, for example, the following [1] to [5].
[1] A production method for a polymer dispersion liquid, the production method comprising:
   a step of preparing a polymer solution containing a polymer in which a crosslinked structure of a water-absorbent resin has been cleaved; and
   a step of bringing a poor solvent for the polymer into contact with the polymer solution to precipitate a solid polymer, thereby obtaining a polymer dispersion liquid in which the solid polymer is dispersed,
   wherein a transmittance of the polymer dispersion liquid at a wavelength of 425 nm and an optical path length of 1 cm is 0.85% or less.
[2] The production method according to [1], wherein the viscosity of the polymer dispersion liquid at 25°C is 5000 mPa·s or less.
[3] The production method according to [1] or [2], wherein the poor solvent includes at least one selected from the group consisting of alcohols having 1 to 10 carbon atoms, phenols, and ketones.
[4] The production method according to any one of [1] to [3], wherein the degree of neutralization of the polymer is 40 mol% or more.
[5] A production method for water-absorbent resin particles, the production method comprising a step of crosslinking the solid polymer in the polymer dispersion liquid obtained by the production method according to any one of [1] to [4] to obtain a water-absorbent resin.

### Advantageous Effects of Invention

According to one aspect of the present invention, it is possible to provide a method for producing a polymer dispersion liquid in which a polymer tends to become true spherical in a dispersion medium in a reverse-phase suspension system.

### Brief Description of Drawings

[Fig. 1] A cross-sectional view showing an example of an absorbent article having an absorbent body.
[Fig. 2] An SEM image of a polymer powder obtained in Example 1.
[Fig. 3] An SEM image of a polymer powder obtained in Comparative Example 1.
[Fig. 4] An SEM image of a polymer powder obtained in Comparative Example 2.
[Fig. 5] An SEM image of a polymer powder obtained in Comparative Example 3.

### Description of Embodiments

The present invention is not limited to the following examples.

In the present specification, "(meth)acryl" means both acrylic and methacrylic. The terms "acrylate" and "methacrylate" are also similarly denoted as "(meth)acrylate". The same also applies to other similar terms. The term "(poly)" includes both cases with and without the "poly" prefix. In the numerical value ranges described in a stepwise manner in the present specification, the upper limit or lower limit of a numerical value range in a certain step can be optionally combined with an upper limit or lower limit of a numerical value range in another step. In the numerical value ranges described in the present specification, an upper limit or lower limit of a numerical value range may be replaced with a value shown in examples. The materials exemplified in the present specification may be used alone or in combination of two or more kinds thereof. "Standard sieve" denotes a test sieve (metal mesh sieve) specified in JIS Z 8801-1:2019. Unless otherwise specified, examples and comparative examples are carried out at 1 atm in a normal-temperature and normal-humidity environment, various parameters disclosed in the present specification are values measured in the same environment, and the temperature of various samples is the normal temperature. "One atmosphere" is 101325 Pa, "normal temperature" is 25°C, and "normal humidity" is 50% RH.

An example of the production method for a spherical polymer includes a step of preparing a polymer solution containing a polymer in which a crosslinked structure of a water-absorbent resin has been cleaved (preparation step), and a step of bringing a poor solvent for the polymer into contact with the polymer solution to precipitate the polymer, thereby obtaining a polymer dispersion liquid in which the solid polymer is dispersed (dispersion step).

### <Water-Absorbent Resin>

The water-absorbent resin may be a water-absorbent resin for recycling. The water-absorbent resin for recycling may be a used water-absorbent resin (that is, a water-absorbent resin in a gel state due to liquid absorption), may be an unused water-absorbent resin (for example, water-absorbent resin waste generated in a step of producing the water-absorbent resin), or may be a mixture of both.

Examples of absorbent articles containing a water-absorbent resin for recycling include diapers (for example, paper diapers), toilet training pants, incontinence pads, sanitary materials (sanitary napkins, tampons, and the like), sweat pads, pet sheets, portable toilet members, and animal excrement treatment materials. The absorbent articles may be used ones. The water-absorbent resin for recycling collected from used water-absorbent articles may form a gel by absorbing liquid during use.

The water-absorbent resin for recycling contains water and a water-absorbent resin. The water-absorbent resin for recycling may contain fibrous substances such as cellulosic fibers and synthetic fibers.

The water-absorbent resin contains, for example, a polymer (crosslinked polymer) of an ethylenically unsaturated monomer. Examples of the ethylenically unsaturated monomer include (meth)acrylic acid and a salt thereof, 2-(meth)acrylamido-2-methylpropanesulfonic acid and a salt thereof, (meth)acrylamide, N,N-dimethyl(meth)acrylamide, 2-hydroxyethyl (meth)acrylate, N-methylol (meth)acrylamide, polyethylene glycol mono(meth)acrylate, N,N-diethylaminoethyl (meth)acrylate, N,N-diethylaminopropyl (meth)acrylate, and diethylaminopropyl (meth)acrylamide. The ethylenically unsaturated monomer may include at least one compound selected from the group consisting of acrylic acid and a salt thereof, methacrylic acid and a salt thereof, acrylamide, methacrylamide, and N,N-dimethylacrylamide.

The crosslinked polymer may contain monomer units other than ethylenically unsaturated monomer units. The proportion of the ethylenically unsaturated monomer units (particularly, (meth)acrylic acid and a salt thereof) in the crosslinked polymer may be 70 mol% to 100 mol% with respect to the total amount of the monomer units. The proportion of (meth)acrylic acid and a salt thereof in the ethylenically unsaturated monomer may be 70 mol% to 100 mol%.

The water-absorbent resin contains, for example, a crosslinked polymer having a poly(meth)acrylic acid structure. Examples of such a crosslinked polymer include a polymer obtained by polymerizing a monomer composition containing (meth)acrylic acid and a crosslinking agent capable of forming a covalent bond by reacting with a carboxyl group of (meth)acrylic acid; a polymer obtained by polymerizing a monomer composition containing (meth)acrylic acid to obtain a polymer and treating a surface of the polymer with a crosslinking agent capable of forming a covalent bond by reacting with a carboxyl group of the polymer; and a polymer obtained by polymerizing a monomer composition containing (meth)acrylic acid and a crosslinking agent capable of forming a covalent bond by reacting with a carboxyl group of (meth)acrylic acid to obtain a polymer and treating a surface of the polymer with a crosslinking agent capable of forming a covalent bond by reacting with a carboxyl group of the polymer.

The shape of the water-absorbent resin for recycling is not particularly limited, and may be an irregular lump shape if it is a used gel, or may be an irregular crushed shape, a scale-like shape, a granular shape, or the like if it is an unused one (generally a dry powder).

### <Preparation Step>

In the preparation step, a polymer solution containing a polymer in which a crosslinked structure of a water-absorbent resin has been cleaved is prepared. In the preparation step, for example, a polymer solution containing a polymer in which a crosslinked structure of a water-absorbent resin has been cleaved is obtained by bringing a water-absorbent resin into contact with a base component. The polymer solution may be a mixture of a polymer (a crosslinked polymer in which the crosslinked structure has been cleaved) and a base component. The polymer solution may or may not contain a water-absorbent resin (a crosslinked polymer in which the crosslinked structure has not been cleaved). In a case where the crosslinked polymer contained in the water-absorbent resin is a crosslinked polymer having a poly(meth)acrylic acid structure, the polymer may be a polymer having a poly(meth)acrylic acid structure, and the polymer solution may be a mixture of a polymer having a poly(meth)acrylic acid structure and a base component.

The base component may be an inorganic base component, and may be, for example, at least one inorganic base component selected from the group consisting of sodium hydroxide, ammonia, potassium hydroxide, and calcium hydroxide, and from the viewpoint of suppressing aggregation of the polymer after the crosslinked structure has been cleaved, may be a monovalent inorganic base component, and may be, for example, at least one monovalent inorganic base component selected from the group consisting of sodium hydroxide, ammonia, and potassium hydroxide.

The polymer solution may be brought into contact with an acid component or the like to adjust the degree of neutralization of the polymer. The degree of neutralization of the polymer means the proportion of neutralized acidic groups (acidic groups forming a salt) with respect to the total amount of acidic groups in the polymer (including neutralized acidic groups). For example, in a case where the polymer is a partially neutralized product of polyacrylic acid, the degree of neutralization of the polymer means the proportion of carboxylate groups with respect to the total amount of carboxyl groups and carboxylate groups contained in the partially neutralized product of polyacrylic acid.

Since the affinity of the polymer for the poor solvent tends to decrease as the degree of neutralization of the polymer increases, from the viewpoint of easily suppressing the amount of the poor solvent used to precipitate the solid polymer, the degree of neutralization of the polymer contained in the polymer solution may be 40 mol% or more, 50 mol% or more, 60 mol% or more, or 70 mol% or more, and from the viewpoint of improving the skin compatibility of the regenerated water-absorbent resin, the degree of neutralization may be 100 mol% or less, 90 mol% or less, 80 mol% or less, or 75 mol% or less. From these viewpoints, the degree of neutralization of the polymer contained in the polymer solution may be 40 mol% to 100 mol%, 50 mol% to 100 mol%, 60 mol% to 100 mol%, or 70 mol% to 100 mol%.

The concentration of the polymer in the polymer solution may be, for example, 0.1% by mass or more, 0.5% by mass or more, or 1% by mass or more, and may be 10% by mass or less, 7% by mass or less, or 5% by mass or less. The concentration of the polymer in the polymer solution may be 0.1% by mass to 10% by mass, 0.5% by mass to 7% by mass, or 1% by mass to 5% by mass.

### <Dispersion Step>

In the dispersion step, a poor solvent for the polymer is brought into contact with the polymer solution to precipitate a solid polymer, thereby obtaining a polymer dispersion liquid in which the solid polymer is dispersed. At this time, the transmittance of the polymer dispersion liquid at a wavelength of 425 nm and an optical path length of 1 cm (hereinafter, may be simply referred to as transmittance) is 0.85% or less. The poor solvent means a solvent in which the solubility of the polymer in 100 g of the solvent at 25°C is less than 0.1 g. The poor solvent may be a water-soluble organic solvent.

When a poor solvent is brought into contact with the polymer solution, the transmittance of the polymer dispersion liquid decreases due to precipitation of the solid polymer. According to the findings of the present inventors, by adjusting the type and/or amount of the poor solvent to be brought into contact so that the transmittance of the polymer dispersion liquid finally obtained is 0.85% or less, a polymer dispersion liquid in which the polymer tends to become true spherical in the dispersion medium in a reverse-phase suspension system can be obtained.

Examples of a method of bringing a poor solvent for the polymer into contact with the polymer solution include a method of adding the poor solvent to the polymer solution and a method of adding the polymer solution to the poor solvent. From the viewpoint of facilitating adjustment of the transmittance at a wavelength of 425 nm and an optical path length of 1 cm, when the poor solvent is added to the polymer solution, the poor solvent may be added to the polymer solution over time. From the same viewpoint, when the polymer solution is added to the poor solvent, the polymer solution may be added to the poor solvent over time. The expression "added over time" denotes that a poor solvent (or a polymer solution) is continuously or intermittently added to a polymer solution (or a poor solvent) over a predetermined time.

Examples of the poor solvent include alcohols having 1 to 10 carbon atoms, phenols, ketones, ethers, and nitriles. From the viewpoint of easily obtaining a spherical polymer with higher purity, the poor solvent may be at least one selected from the group consisting of alcohols having 1 to 10 carbon atoms, phenols, and ketones.

Examples of the alcohols having 1 to 10 carbon atoms include methanol, ethanol, propanol, butanol, pentanol, ethylene glycol, and glycerin. Examples of the phenols include phenol, cresol, dibutylhydroxytoluene, and eugenol. Examples of the ketones include acetone, methyl ethyl ketone, and diethyl ketone. Examples of the ethers include dimethyl ether, ethyl methyl ether, diethyl ether, furan, and tetrahydrofuran. Examples of the nitriles include acrylonitrile. The poor solvent may be at least one selected from the group consisting of methanol, ethanol, and acetone.

The concentration of the poor solvent in the polymer dispersion liquid finally obtained may be 60% by mass or less, 55% by mass or less, or 50% by mass or less from the viewpoint of suppressing an increase in the transmittance of the polymer dispersion liquid due to generation of aggregated precipitates caused by excessive precipitation of the polymer. The concentration of the poor solvent in the polymer dispersion liquid may be 10% by mass or more or 20% by mass or more from the viewpoint of precipitating a sufficient amount of the polymer. The concentration of the poor solvent in the polymer dispersion liquid may be 10% by mass to 60% by mass or 20% by mass to 55% by mass.

The transmittance of the polymer dispersion liquid at a wavelength of 425 nm and an optical path length of 1 cm is 0.85% or less, and from the viewpoint of sufficiently precipitating a solid polymer that tends to form true spherical particles in the dispersion medium in a reverse-phase suspension system, the transmittance may be 0.80% or less, 0.70% or less, or less than 0.70%. The transmittance of the polymer dispersion liquid at a wavelength of 425 nm and an optical path length of 1 cm may be 0.01% or more, 0.10% or more, 0.20% or more, or 0.30% or more from the viewpoint of facilitating the formation of true spherical particles in the dispersion medium in a reverse-phase suspension system by suppressing aggregation of the precipitated solid polymer. From these viewpoints, the transmittance of the polymer dispersion liquid may be 0.01% to 0.80%, 0.01% to 0.70%, or 0.01% or more and less than 0.70%. The transmittance of the polymer dispersion liquid at a wavelength of 425 nm and an optical path length of 1 cm is measured by the method described in the Examples below.

The viscosity of the polymer dispersion liquid at 25°C may be 5000 mPa·s or less, 3000 mPa·s or less, or 2000 mPa·s or less from the viewpoint of suppressing aggregation of the solid polymer in the dispersion medium by facilitating dispersion in the dispersion medium in a reverse-phase suspension system, and thereby facilitating the formation of true spherical particles. The viscosity of the polymer dispersion liquid at 25°C may be 10 mPa·s or more, 50 mPa·s or more, 100 mPa·s or more, 500 mPa·s or more, 1000 mPa·s or more, 1500 mPa·s or more, 3000 mPa·s or more, 5000 mPa·s or more, or 10000 mPa·s or more. From these viewpoints, the viscosity of the polymer dispersion liquid at 25°C may be 50 mPa·s to 5000 mPa·s, 50 mPa·s to 3000 mPa·s, or 50 mPa·s to 2000 mPa·s, 100 mPa·s to 5000 mPa·s, 100 mPa·s to 3000 mPa·s, or 100 mPa·s to 2000 mPa·s. The viscosity of the polymer dispersion liquid at 25°C is measured by the method described in the Examples below.

The median particle diameter of the dried product of the solid polymer after the polymer dispersion liquid is added to the dispersion medium in a reverse-phase suspension system may be 100 µm or more, 150 µm or more, or 200 µm or more, and may be less than 300 µm or 290 µm or less, and may be 100 µm or more and less than 300 µm. The median particle diameter of the solid polymer is measured by the method described in the Examples below (method for measuring the median particle diameter of polymer powder).

### <Crosslinking Step>

Since the polymer dispersion liquid tends to become true spherical in the dispersion medium in a reverse-phase suspension system, the polymer dispersion liquid can be suitably used for reverse-phase suspension polymerization. Therefore, after the polymer dispersion liquid is obtained, the solid polymer in the obtained polymer dispersion liquid may be crosslinked to obtain a water-absorbent resin. That is, another embodiment of the present invention is a production method for water-absorbent resin particles, the production method including a step of crosslinking the solid polymer in the polymer dispersion liquid obtained by the above-described production method to obtain a water-absorbent resin (crosslinking step).

After the solid polymer is obtained and before the crosslinking step is carried out, dilution, heating, addition of a salt (a salt containing a monovalent cation), or the like may be performed on the polymer dispersion liquid from the viewpoint of reducing the viscosity of the polymer dispersion liquid. In addition, after the solid polymer is obtained and before the solid polymer is crosslinked, a sterilization treatment may be performed on the solid polymer.

In the crosslinking step, for example, a solution containing a crosslinking agent capable of forming a covalent bond with a functional group contained in the solid polymer is prepared, the solution containing the crosslinking agent is added to the polymer dispersion liquid, and the solid polymer is crosslinked through a covalent bond to obtain a crosslinked polymer. In the crosslinking step, the polymer dispersion liquid may undergo gelation. In addition, after the polymer dispersion liquid is mixed with a dispersion medium containing a poor solvent other than the poor solvent used in obtaining the polymer dispersion liquid, the mixture may be mixed with a solution containing a crosslinking agent to crosslink the spherical polymer to obtain a crosslinked polymer. In addition, after a crosslinking agent is added to a dispersion medium containing a poor solvent other than the poor solvent used in obtaining the polymer dispersion liquid, the dispersion medium may be mixed with the polymer dispersion liquid to crosslink the spherical polymer to obtain a crosslinked polymer. By crosslinking the solid polymer, the entire polymer dispersion liquid loses fluidity, and a gel containing a crosslinked polymer and water is formed. In a case where the crosslinking proceeds to the extent that a gel is formed, it is particularly easy to obtain water-absorbent resin particles having high water absorption performance and a small amount of soluble content.

Examples of the functional group contained in the solid polymer include a carboxyl group. In a case where the solid polymer has a carboxyl group, the solid polymer is crosslinked by a reaction between the carboxyl group and a crosslinking agent and/or a reaction between the carboxyl groups. The covalent bond may be at least one selected from the group consisting of an ester bond, a thioester bond, an amide bond, an ether bond, and a carbon-carbon bond. The spherical polymer may be crosslinked through, for example, at least one group selected from the group consisting of a carboxylic acid ester group, a thioester group, an amide group, an acid anhydride group, an oxyalkylene group, and an oxyarylene group.

Examples of the crosslinking agent include aliphatic polyhydric alcohols such as (poly)ethylene glycol, (poly)propylene glycol, (poly)glycerin, and pentaerythritol; glycidyl compounds such as (poly)ethylene glycol diglycidyl ether, (poly)propylene glycol diglycidyl ether, and (poly)glycerin diglycidyl ether; bisacrylamide compounds such as N,N'-methylene bis(meth)acrylamide; allylated starch; diallyl phthalate; N,N',N''-triallylisocyanurate; divinylbenzene; ethylenediamine, polyethyleneimine, and glycidyl (meth)acrylate.

The amount of the crosslinking agent, per 100 parts by mass of the solid polymer, may be 0.0001 parts by mass or more, 0.001 parts by mass or more, or 0.005 parts by mass or more, and may be 10 parts by mass or less, 10 parts by mass or less, or 5 parts by mass or less. The amount of the crosslinking agent may be 0.0001 parts by mass to 15 parts by mass, 0.001 parts by mass to 10 parts by mass, or 0.005 parts by mass to 5 parts by mass per 100 parts by mass of the solid polymer.

The reaction temperature for crosslinking the solid polymer is appropriately set depending on the kind, the amount, and the like of the crosslinking agent to be used, but may be 50°C or higher, 80°C or higher, or 100°C or higher, and may be 220°C or lower, 200°C or lower, or 180°C or lower. The reaction temperature for crosslinking the solid polymer may be 50°C to 220°C, 80°C to 200°C, or 100°C to 180°C.

The reaction time for crosslinking the solid polymer is appropriately set depending on the kind and amount of the crosslinking agent to be used, the reaction temperature, and the like, but may be 1 minute to 200 minutes or 5 minutes to 150 minutes.

After the crosslinked polymer is obtained through the crosslinking step, a surface crosslinking step of performing surface crosslinking of the crosslinked polymer may be carried out. The surface crosslinking can be performed, for example, by adding a crosslinking agent for performing the surface crosslinking (surface crosslinking agent) to the crosslinked polymer and carrying out the reaction.

The surface crosslinking agent may be a compound having two or more reactive functional groups having reactivity with the crosslinked polymer. The surface crosslinking agent may be the same as or different from the crosslinking agent in the crosslinking step.

The reactive functional group of the surface crosslinking agent may be a carbonate group, an alcoholic hydroxy group, an epoxy group, a halogeno group in a haloepoxy compound, an isocyanate group, an oxetanyl group, an oxazoline group, or a combination thereof. The carbonate group can react with two other molecules, and thus is regarded as two reactive functional groups.

Examples of the surface crosslinking agent containing a carbonate group include alkylene carbonate (ethylene carbonate and the like). Examples of surface crosslinking agents containing an alcoholic hydroxyl group include polyol compounds such as ethylene glycol, propylene glycol, 1,4-butanediol, trimethylolpropane, glycerin, polyoxyethylene glycol, polyoxypropylene glycol, and polyglycerin, and hydroxyalkylamide compounds (bis[N,N-di(β-hydroxyethyl)]adipamide and the like). Examples of the surface crosslinking agent containing two or more epoxy groups include (poly)ethylene glycol diglycidyl ether, (poly)glycerin diglycidyl ether, (poly)glycerin triglycidyl ether, trimethylolpropane triglycidyl ether, (poly)propylene glycol polyglycidyl ether, and (poly)glycerol polyglycidyl ether. Examples of haloepoxy compounds containing an epoxy group and a halogeno group include epichlorohydrin, epibromohydrin, and α-methylepichlorohydrin. Examples of the surface crosslinking agent containing an isocyanate group include 2,4-tolylene diisocyanate and hexamethylene diisocyanate. Examples of surface crosslinking agents containing an oxetanyl group include 3-methyl-3-oxetane methanol, 3-ethyl-3-oxetane methanol, 3-butyl-3-oxetane methanol, 3-methyl-3-oxetane ethanol, 3-ethyl-3-oxetane ethanol, and 3-butyl-3-oxetane ethanol.

The amount of the surface crosslinking agent may be 0.001 parts by mass or more, 0.005 parts by mass or more, or 0.01 parts by mass or more, and may be 5 parts by mass or less, 3 parts by mass or less, or 1 part by mass or less per 100 parts by mass of the crosslinked polymer. The amount of the crosslinking agent may be 0.001 parts by mass to 5 parts by mass, 0.005 parts by mass to 3 parts by mass, or 0.01 parts by mass to 1 part by mass per 100 parts by mass of the crosslinked polymer.

When the polymer dispersion liquid is mixed with a dispersion medium, the dispersion medium used is a liquid mainly composed of a hydrophobic substance, and for example, the dispersion medium is a hydrocarbon-based dispersion medium. Examples of the hydrocarbon-based dispersion medium include chain aliphatic hydrocarbons such as n-hexane, n-heptane, 2-methylhexane, 3-methylhexane, 2,3-dimethylpentane, 3-ethylpentane, and n-octane; alicyclic hydrocarbons such as cyclohexane, methylcyclohexane, cyclopentane, methylcyclopentane, trans-1,2-dimethylcyclopentane, cis-1,3-dimethylcyclopentane, and trans-1,3-dimethylcyclopentane; and aromatic hydrocarbons such as benzene, toluene, and xylene. The hydrocarbon-based dispersion medium may be used alone or in combination of two or more kinds thereof.

The dispersion medium may contain a polymeric dispersant, a surfactant, or the like from the viewpoint of improving the dispersibility of the solid polymer.

Examples of the polymeric dispersant include hydrophilic polymeric dispersants such as polyvinyl alcohol (PVA), polyvinylpyrrolidone (PVP), polyethylene glycol (PEG), polypropylene glycol, polyethylene glycol/polypropylene glycol block copolymer, polyglycerin, polyoxyethylene glycerin, polyoxypropylene glycerin, polyoxyethylene/polyoxypropylene glycerin copolymer, and polyoxyethylene sorbitan fatty acid ester; and hydrophobic polymeric dispersants such as maleic anhydride-modified polyethylene, maleic anhydride-modified polypropylene, maleic anhydride-modified ethylene-propylene copolymer, maleic anhydride-modified EPDM (ethylene-propylene-diene terpolymer), maleic anhydride-modified polybutadiene, maleic anhydride/ethylene copolymer, maleic anhydride/propylene copolymer, maleic anhydride/ethylene/propylene terpolymer, maleic anhydride/butadiene copolymer, polyethylene, polypropylene, ethylene-propylene copolymer, oxidized polyethylene, oxidized polypropylene, oxidized ethylene-propylene copolymer, ethylene-acrylic acid copolymer, ethyl cellulose, and ethyl hydroxyethyl cellulose.

Examples of the surfactant include nonionic surfactants such as sorbitan fatty acid ester, (poly)glycerin fatty acid ester, sucrose fatty acid ester, polyoxyethylene sorbitan fatty acid ester, polyoxyethylene glycerin fatty acid ester, sorbitol fatty acid ester, polyoxyethylene sorbitol fatty acid ester, polyoxyethylene alkyl ether, polyoxyethylene alkylphenyl ether, polyoxyethylene castor oil, polyoxyethylene hydrogenated castor oil, alkylallyl formaldehyde condensed polyoxyethylene ether, polyoxyethylene polyoxypropylene block copolymer, polyoxyethylene polyoxypropyl alkyl ether, and polyethylene glycol fatty acid ester; and anionic surfactants such as fatty acid salt, alkylbenzene sulfonate, alkylmethyl taurate, polyoxyethylene alkylphenyl ether sulfate ester salt, polyoxyethylene alkyl ether sulfonate, polyoxyethylene alkyl ether phosphate ester salt, and polyoxyethylene alkylallyl ether phosphate ester salt.

In a reverse-phase suspension system, when the polymer dispersion liquid is mixed with a dispersion medium, the volume ratio of the polymer dispersion liquid to the dispersion medium (polymer dispersion liquid/dispersion medium) may be 0.1 or more, 0.15 or more, 0.3 or more, or 0.4 or more from the viewpoint that the median particle diameter of the solid polymer in the dispersion medium tends to become large (that is, the median particle diameter of the crosslinked polymer to be obtained can be expected to become large). The volume ratio of the polymer dispersion liquid to the dispersion medium (polymer dispersion liquid/dispersion medium) may be 0.7 or less, 0.6 or less, or 0.55 or less from the viewpoint of facilitating crosslinking in the reverse-phase suspension system. The volume ratio of the polymer dispersion liquid to the dispersion medium (polymer dispersion liquid/dispersion medium) may be 0.1 to 0.7, 0.15 to 0.6, or 0.15 to 0.55.

A regenerated water-absorbent resin containing a crosslinked polymer is formed by a method including removing water from the crosslinked polymer obtained through any optional surface crosslinking step. In a case where the reaction solution subjected to the crosslinking step is an aqueous solution, the water-absorbent resin particles are formed by a method including a drying step of removing water from a lumpy crosslinked polymer formed by gelation of the reaction solution itself and drying the crosslinked polymer to form a dried product, and a pulverization step of pulverizing the dried product.

A method of drying the crosslinked polymer may be, for example, a typical method such as a pressing method such as centrifugation, dehydration using an organic solvent, natural drying, heat drying, air drying, freeze drying, or a combination thereof. From the viewpoint of efficiently removing water, the heating temperature for drying may be 80°C to 220°C, 90°C to 200°C, or 100°C to 180°C.

The moisture content of the dried product may be, for example, 20% by mass or less, 10% by mass or less, or 5% by mass or less. The moisture content of the dried product means the proportion of moisture in the dried product based on the total amount of the dried product. The moisture content of the dried product can be determined as the difference in mass of the crosslinked polymer before and after heating, when the crosslinked polymer containing water is heated at 200°C for 2 hours.

Before drying the crosslinked polymer, a coarsely crushed product including structures of a somewhat small size may be formed by coarsely crushing the crosslinked polymer. Water can be efficiently removed by forming the coarsely crushed product. The structures constituting the coarsely crushed product can be, for example, elongated structures, granular structures (particles), or a combination thereof. The coarsely crushed product may include a plurality of structures having a shape that enables them to pass through a circular hole having a diameter of 10 mm or 7 mm. The elongated structure may be bent, and in a case where the maximum width thereof is 10 mm or less, it can be said that the elongated structure has a shape that enables the structure to pass through a circular hole having a diameter of 10 mm. The granular structure (particle) may be irregularly shaped and may have a shape that enables the structure to pass through a circular hole having a diameter of 10 mm while changing the orientation. Examples of a coarse crusher for coarsely crushing the crosslinked polymer include a kneader (for example, a pressure type kneader or a double-arm type kneader), a meat chopper, a cutter mill, and a pharma mill.

The dried product is pulverized to form water-absorbent resin particles. The pulverization method is not particularly limited. For example, the dried product can be pulverized using a pulverizer such as a centrifugal pulverizer, a roller mill, a stamp mill, a jet mill, a high-speed rotary pulverizer, a container drive type mill, or the like.

The water-absorbent resin particles obtained by pulverization may be classified. The classification denotes an operation of dividing a particle group (powder) into two or more particle groups having different particle size distributions. Some of the water-absorbent resin particles after the classification may be pulverized and classified again.

The classification method is not particularly limited, but may be, for example, screen classification or airflow classification. The screen classification is a method of classifying particles on a screen into particles that pass through the openings of the screen and particles that do not pass through the openings by vibrating the screen. The screen classification can be performed by using, for example, a vibrating screen, a rotary sifter, a cylindrical stirring screen, a blower sifter, or a Ro-tap shaker. The airflow classification is a method of classifying particles using the flow of air.

The median particle diameter of the powder of the crosslinked polymer obtained by performing pulverization and, as necessary, classification may be, for example, 200 µm to 500 µm or 200 µm to 500 µm. The particle size distribution may be adjusted by mixing two or more powders having different median particle diameters, which are obtained by the classification. The median particle diameter of the powder of the crosslinked polymer can be adjusted by the mixing ratio of the polymer dispersion liquid and the dispersion medium, the addition of a surfactant, and the like.

### <Absorbent Article>

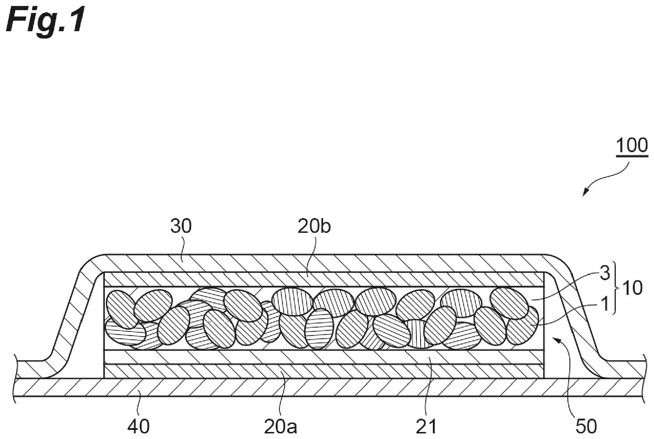

Fig. 1 is a cross-sectional view showing an example of an absorbent article having an absorbent body containing water-absorbent resin particles. An absorbent article 100 shown in Fig. 1 includes a water-absorbent sheet 50 having a film-like absorbent body 10, a liquid permeable sheet 30, and a liquid impermeable sheet 40.

The water-absorbent sheet 50 has an absorbent body 10 containing a powder of water-absorbent resin particles 1 and two core wrap sheets 20a and 20b. The absorbent body 10 is disposed inside the core wrap sheets 20a and 20b. The shape of the absorbent body 10 is retained by being sandwiched between the two core wrap sheets 20a and 20b. The core wrap sheets 20a and 20b may be two sheets, a single folded sheet, or a single bag body. A sheet member having no other constituent members provided on the outside of the core wrap sheets 20a and 20b that wrap the absorbent body 10 is also particularly referred to as a water-absorbent sheet.

The absorbent body 10 is a constituent member which mainly contains a powder of the water-absorbent resin particles 1 and whose shape is retained to be constant. The absorbent body 10 may or may not include a fibrous substance 3 in addition to the powder of the water-absorbent resin particles 1. The content of the water-absorbent resin particles 1 in the absorbent body 10 may be 50% by mass or more and 100% by mass or less, 60% by mass or more and 100% by mass or less, 70% by mass or more and 100% by mass or less, 80% by mass or more and 100% by mass or less, or 90% by mass or more and 100% by mass or less with respect to the mass of the absorbent body 10.

The thickness of the absorbent body 10 may be, for example, 20 mm or less, 15 mm or less, 10 mm or less, 5 mm or less, 4 mm or less, or 3 mm or less, or may be 0.1 mm or more or 0.3 mm or more. The thickness of the absorbent body 10 may be 0.1 mm or more and 20 mm or less. The mass of the absorbent body 10 per unit area may be 1000 g/m² or less, 800 g/m² or less, or 600 g/m² or less, or may be 100 g/m² or more.

The fibrous substance 3 can be, for example, cellulosic fibers, synthetic fibers, or a combination thereof. Examples of the cellulosic fibers include pulverized wood pulp, cotton, cotton linter, rayon, and cellulose acetate. Examples of the synthetic fibers include polyamide fibers, polyester fibers, and polyolefin fibers. The fibrous substance may be hydrophilic fibers (for example, pulp).

The absorbent body 10 may further contain an inorganic powder (for example, amorphous silica), a deodorant, an antibacterial agent, a fragrance, or the like. In a case where the water-absorbent resin particles 1 include inorganic particles, the absorbent body 10 may contain an inorganic powder in addition to the inorganic particles in the water-absorbent resin particles 1.

The water-absorbent sheet 50 may further have an adhesive 21 interposed between the core wrap sheet 20a and the absorbent body 10. An adhesive layer may be interposed between the core wrap sheets 20a and 20b on both sides and the absorbent body 10. The adhesive 21 is not particularly limited, and may be, for example, a hot melt adhesive.

The core wrap sheets 20a and 20b may be, for example, non-woven fabrics. The two core wrap sheets 20a and 20b may be the same or different non-woven fabrics. The non-woven fabrics may be non-woven fabrics (short-fiber non-woven fabrics) formed of short fibers (that is, staple fibers), or may be non-woven fabrics (long-fiber non-woven fabrics) formed of long fibers (that is, filaments). Although not limited thereto, the staples generally may have a fiber length of several hundred mm or less.

The core wrap sheets 20a and 20b may be thermal bonded non-woven fabrics, air-through non-woven fabrics, resin bonded non-woven fabrics, spunbond non-woven fabrics, melt-blown non-woven fabrics, air-laid non-woven fabrics, spunlace non-woven fabrics, point-bonded non-woven fabrics, or a laminate containing two or more non-woven fabrics selected from these non-woven fabrics.

The non-woven fabric used as the core wrap sheets 20a and 20b can be a non-woven fabric formed of synthetic fibers, natural fibers, or a combination thereof. Examples of the synthetic fibers include fibers containing synthetic resins selected from polyolefin such as polyethylene (PE) and polypropylene (PP), polyester such as polyethylene terephthalate (PET), polytrimethylene terephthalate (PTT), and polyethylene naphthalate (PEN), polyamide such as nylon, and rayon. Examples of the natural fibers include fibers containing cotton, silk, hemp, or pulp (cellulose). The fibers forming the non-woven fabrics may be polyolefin fibers, polyester fibers, or a combination thereof. The core wrap sheets 20a and 20b may be tissue paper.

The water-absorbent sheet 50 may be used for producing various other absorbent articles. Examples of the absorbent articles include diapers (for example, paper diapers), toilet training pants, incontinence pads, sanitary materials (sanitary napkins, tampons, and the like), sweat pads, pet sheets, portable toilet members, and animal excrement treatment materials. The absorbent bodies constituting these absorbent articles frequently move or deform due to the movement or the like of a user of the absorbent article.

The liquid permeable sheet 30 is disposed at the position of the outermost layer on a side where the liquid to be absorbed enters. The liquid permeable sheet 30 is disposed outside the core wrap sheet 20b in a state of being in contact with the core wrap sheet 20b. The liquid impermeable sheet 40 is disposed at the position of the outermost layer on the side opposite to the liquid permeable sheet 30 in the absorbent article 100. The liquid impermeable sheet 40 is disposed outside the core wrap sheet 20a in a state of being in contact with the core wrap sheet 20a. The liquid permeable sheet 30 and the liquid impermeable sheet 40 have main surfaces wider than the main surface of the water-absorbent sheet 50, and outer edges of the liquid permeable sheet 30 and the liquid impermeable sheet 40 extend around the absorbent body 10 and the core wrap sheets 20a and 20b. However, the size relationship between the absorbent body 10, the core wrap sheets 20a and 20b, the liquid permeable sheet 30, and the liquid impermeable sheet 40 is not particularly limited, and is appropriately adjusted depending on the applications of the absorbent article or the like.

The liquid permeable sheet 30 may be a non-woven fabric. The non-woven fabric used as the liquid permeable sheet 30 may have appropriate hydrophilicity from the viewpoint of the liquid absorption performance of the absorbent article. From this viewpoint, the liquid permeable sheet 30 may be a non-woven fabric having a hydrophilicity of 5 to 200, which is measured in accordance with the measuring method of the Japan Technical Association of Pulp and Paper Industry Test Method No. 68 (2000). The hydrophilicity of the non-woven fabric may be 10 to 150. For the details of Pulp and Paper Test Method No. 68, reference can be made to, for example, WO2011/086843.

The non-woven fabric having hydrophilicity may be formed of fibers such as rayon fibers exhibiting an appropriate hydrophilicity, or may be formed of fibers obtained by performing a hydrophilic treatment on hydrophobic chemical fibers such as polyolefin fibers and polyester fibers. Examples of a method of obtaining a non-woven fabric including hydrophobic chemical fibers that have been subjected to a hydrophilic treatment include a method of obtaining a non-woven fabric by a spunbond method using a mixture obtained by mixing a hydrophilic agent with hydrophobic chemical fibers, a method of incorporating a hydrophilic agent in preparing a spunbond non-woven fabric with hydrophobic chemical fibers, and a method of impregnating a spunbond non-woven fabric obtained using hydrophobic chemical fibers with a hydrophilic agent. As the hydrophilic agent, an anionic surfactant such as an aliphatic sulfonate or a higher alcohol sulfate ester salt, a cationic surfactant such as a quaternary ammonium salt, a nonionic surfactant such as polyethylene glycol fatty acid ester, polyglycerin fatty acid ester, or sorbitan fatty acid ester, a silicone surfactant such as polyoxyalkylene-modified silicone, and a stain release agent formed of a polyester-based, polyamide-based, acrylic-based, or urethane-based resin, or the like are used.

From the viewpoint of imparting satisfactory liquid permeability, flexibility, strength, and cushioning properties to the absorbent article and from the viewpoint of accelerating the liquid permeation rate of the absorbent article, the basis weight (mass per unit area) of the non-woven fabric used as the liquid permeable sheet 30 may be 5 g/m² to 200 g/m², 8 g/m² to 150 g/m², or 10 g/m² to 100 g/m². The thickness of the liquid permeable sheet 30 may be 20 µm to 1400 µm, 50 µm to 1200 µm, or 80 µm to 1000 µm.

The liquid impermeable sheet 40 prevents a liquid absorbed by the absorbent body 10 from leaking to the outside from the liquid impermeable sheet 40 side. The liquid impermeable sheet 40 may be a resin sheet or a non-woven fabric. The resin sheet may be a sheet formed of a synthetic resin such as polyethylene, polypropylene, polyvinyl chloride, or the like. The non-woven fabric may be a spunbond/melt-blown/spunbond (SMS) non-woven fabric in which a waterproof melt-blown non-woven fabric is sandwiched between high-strength spunbond non-woven fabrics. The liquid impermeable sheet 40 may be a composite sheet of a resin sheet and a non-woven fabric (for example, a spunbond non-woven fabric, a spunlace non-woven fabric). The liquid impermeable sheet 40 may have breathability from the viewpoints that dampness generated at the time of wearing is reduced, and thereby discomfort to a wearer can be reduced. As the liquid impermeable sheet 40 having breathability, it is possible to use, for example, a sheet of low-density polyethylene (LDPE) resin.

From the viewpoint of ensuring the flexibility so that the feeling of wearing the absorbent article is not impaired, the basis weight (mass per unit area) of the liquid impermeable sheet 40 may be 10 g/m² to 50 g/m².

### Examples

Hereinafter, the present invention will be described in more detail with reference to the Examples.

### <Evaluation Method>

The transmittance, viscosity, shape, and median particle diameter were measured according to the following evaluation methods. The measurement results are as shown in Table 1.

### [Transmittance]

A spectrophotometer manufactured by Shimadzu Corporation (model number: UV-1850) was used for the measurement of transmittance. The polymer dispersion liquid was placed in a cell for UV measurement (optical path length: 1 cm), and defoamed by operating a tabletop centrifuge manufactured by Kokusan (model number: H-36) at 2000 rpm for 1 minute. In a case where defoaming was not complete, the same operation was performed using the tabletop centrifuge to defoam. Next, the cell was set in the spectrophotometer, and the transmittance was measured at a measurement wavelength of 425 nm. For the measurement of transmittance, calibration was performed so that the transmittance of distilled water was 100%.

### [Viscosity]

The polymer dispersion liquid was placed in a 100 mL centrifuge tube and degassed using a centrifuge (manufactured by Kokusan Co., Ltd., tabletop centrifuge H-36). Using a B-type viscometer (manufactured by Brookfield, model number: DV1MRVTJ0), the viscosity at 25°C after 1 minute was measured at a rotation speed of 20 rpm. The rotor used for the measurement was rotor No. 4 when the viscosity was less than 8000 mPa·s, rotor No. 6 when the viscosity was 10000 mPa·s to 40000 mPa·s, and rotor No. 7 when the viscosity was 40000 mPa·s or more.

### [Shape]

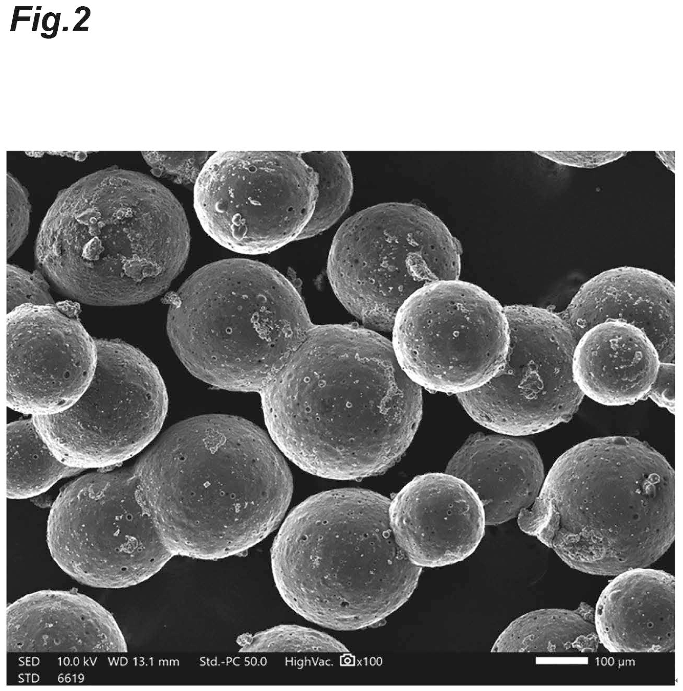

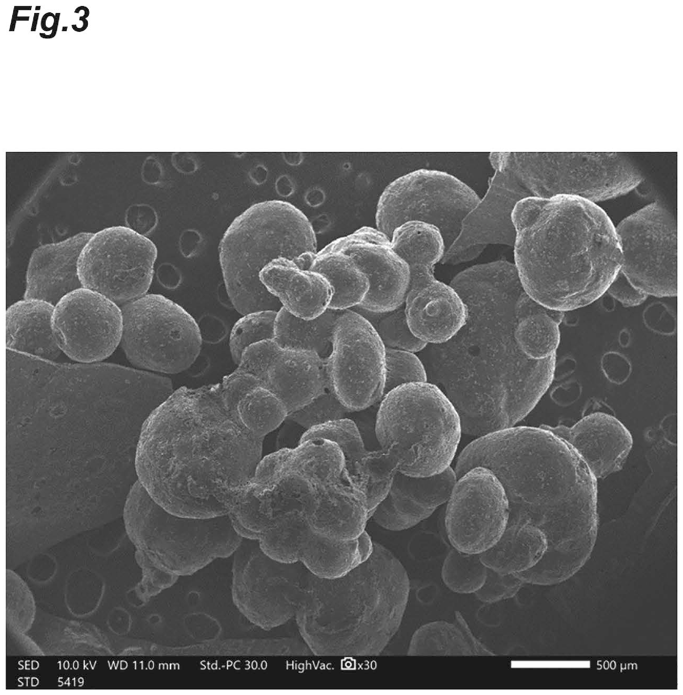

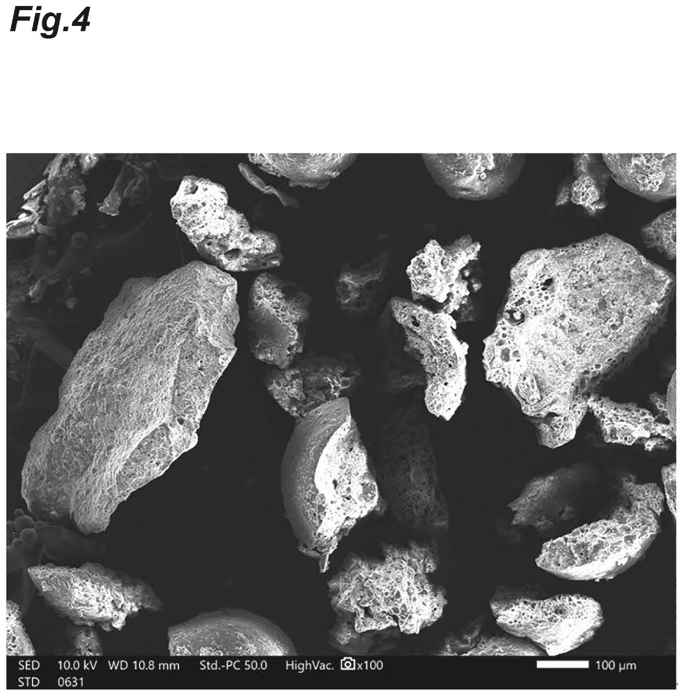

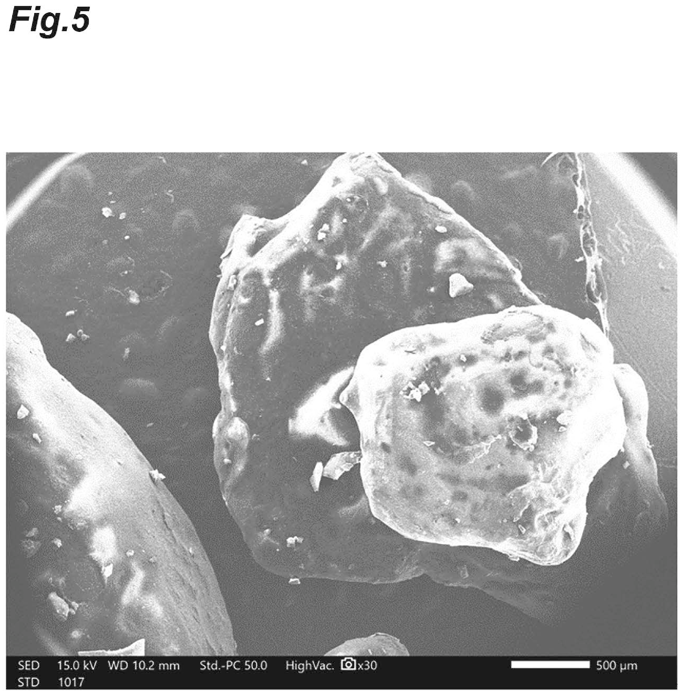

The shape of the polymer powder was observed using an SEM (scanning electron microscope). The SEM image of the polymer powder obtained in Example 1 is shown in Fig. 2, the SEM image of the polymer powder obtained in Comparative Example 1 is shown in Fig. 3, the SEM image of the polymer powder obtained in Comparative Example 2 is shown in Fig. 4, and the SEM image of the polymer powder obtained in Comparative Example 3 is shown in Fig. 5.

### [Median Particle Diameter (Robot Sifter Method)]

The median particle diameter of the polymer powder was measured using an automatic particle size distribution analyzer of the sonic sieving type (Robot Sifter manufactured by Seishin Enterprise Co., Ltd., RPS-01). The measurement conditions were as follows: sonic intensity: 50 Lv, frequency: 51 Hz, classification time: 5 min, sweep time: 0.1 min, and pulse interval: 1 second. The openings of the standard sieves used for the measurement were 710, 400, 150, 106, 75, 63, 45, and 38 µm.

### (Example 1)

94 g of ion-exchanged water was charged into a 500 mL polybeaker equipped with a stirrer having four inclined paddle blades with a diameter of 7.5 cm. While stirring the solution at 400 rpm at normal temperature, 6 g of an uncrosslinked partially neutralized polyacrylic acid (manufactured by Sumitomo Seika Chemicals Co., Ltd., Aquperna AP-70) was added. When the solution became viscous and winding around the stirring shaft was observed, the stirring speed was reduced to 100 rpm, and the solution was stirred for 6 hours to dissolve, thereby preparing a 6 mass% sodium polyacrylate aqueous solution (degree of neutralization: 70 mol%) as a polymer solution. While stirring the obtained polymer solution (100 g) at 1000 rpm using the above-described stirrer, 100 g of a 70 mass% ethanol aqueous solution as a poor solvent was added dropwise at 10 mL/min using a liquid delivery pump, thereby preparing a polymer dispersion liquid in which a solid polymer had precipitated. 300 g of normal heptane as a hydrocarbon dispersion medium was weighed into a 2000 mL separable flask equipped with a stirrer having four inclined paddle blades with a diameter of 5.0 cm, a reflux condenser, and a nitrogen tube, and 0.1 g of a maleic anhydride-modified ethylene-propylene copolymer (trade name: Hi-WAX 1105A, manufactured by Mitsui Chemicals, Inc.) as a polymeric dispersant and 0.1 g of sucrose stearic acid ester (trade name: Ryoto Sugar Ester S-370, HLB value 3, manufactured by Mitsubishi Chemical Foods Corporation) as a surfactant were added, and the mixture was heated and dissolved while stirring at 500 rpm, and then allowed to cool to 70°C. Thereafter, the stirring speed was changed to 1000 rpm, and the polymer dispersion liquid (200 g) was added using a funnel. The volume ratio of the polymer dispersion liquid to the dispersion medium (polymer dispersion liquid/dispersion medium) was 0.50. After holding at 70°C for 30 minutes, the polymer dispersion liquid was heated using an oil bath at 125°C to remove the dispersion medium and solvent, thereby obtaining a powder. The obtained powder was classified using a standard sieve with an opening of 300 µm to obtain a polymer powder (crosslinked polymer).

### (Example 2)

A polymer powder (crosslinked polymer) was obtained by performing the same operation as in Example 1, except that a 98 mass% methanol aqueous solution was used as the poor solvent and the holding temperature after adding the polymer dispersion liquid to the liquid containing the hydrocarbon dispersion medium was changed to 60°C.

### (Example 3)

A polymer powder (crosslinked polymer) was obtained by performing the same operation as in Example 1, except that a 70 mass% isopropyl alcohol aqueous solution was used as the poor solvent.

### (Example 4)

A polymer powder (crosslinked polymer) was obtained by performing the same operation as in Example 1, except that an 80 mass% acetone aqueous solution was used as the poor solvent, the holding temperature after adding the polymer dispersion liquid to the liquid containing the hydrocarbon dispersion medium was changed to 60°C, and the opening of the standard sieve used for classifying the obtained powder was changed to 850 µm.

### (Example 5)

A polymer powder (crosslinked polymer) was obtained by performing the same operation as in Example 1, except that the amount of ion-exchanged water was changed to 96 g, the amount of the uncrosslinked partially neutralized polyacrylic acid was changed to 4 g, and an 80 mass% ethanol aqueous solution was used as the poor solvent.

### (Example 6)

A polymer powder (crosslinked polymer) was obtained by performing the same operation as in Example 1, except that the uncrosslinked partially neutralized polyacrylic acid was changed to Aquperna AP-40F manufactured by Sumitomo Seika Chemicals Co., Ltd. to prepare a 6 mass% sodium polyacrylate aqueous solution (degree of neutralization: 40 mol%) as a polymer solution, an 80 mass% ethanol aqueous solution was used as the poor solvent, and the opening of the standard sieve used for classifying the obtained powder was changed to 850 µm.

### (Example 7)

A polymer powder (crosslinked polymer) was obtained by performing the same operation as in Example 1, except that the amount of ion-exchanged water was changed to 90.67 g, 3.33 g of a 30 mass% sodium hydroxide aqueous solution was added to the uncrosslinked partially neutralized polyacrylic acid to prepare a sodium polyacrylate aqueous solution with a degree of neutralization of 100 mol% as a polymer solution, a 42 mass% ethanol aqueous solution was used as the poor solvent, and the opening of the standard sieve used for classifying the obtained powder was changed to 850 µm.

### (Example 8)

A polymer powder (crosslinked polymer) was obtained by performing the same operation as in Example 1, except that the volume ratio of the polymer dispersion liquid to the dispersion medium (polymer dispersion liquid/dispersion medium) was changed to 0.15.

### (Example 9)

A polymer powder (crosslinked polymer) was obtained by performing the same operation as in Example 1, except that the volume ratio of the polymer dispersion liquid to the dispersion medium (polymer dispersion liquid/dispersion medium) was changed to 0.30.

### (Comparative Example 1)

The same operation as in Example 1 was performed, except that the amount of ion-exchanged water was changed to 194 g, the sodium polyacrylate aqueous solution (polymer solution) was mixed with normal heptane as-is without adding a poor solvent, and the obtained polymer could not be classified because its shape was too large.

### (Comparative Example 2)

The same operation as in Example 1 was performed, except that a 60 mass% ethanol aqueous solution was used as the poor solvent, and as a result, an aggregate of the polymer was obtained. The obtained aggregate was irregularly shaped.

### (Comparative Example 3)

The same operation as in Example 1 was performed, except that the amount of ion-exchanged water was changed to 171 g and the amount of the uncrosslinked partially neutralized polyacrylic acid was changed to 19 g, to prepare a 10 mass% sodium polyacrylate aqueous solution (degree of neutralization: 70 mol%) as a polymer solution. Next, while stirring the obtained polymer solution at 1000 rpm using the above-described stirrer, 10 g of a 100 mass% ethanol aqueous solution as a poor solvent was added dropwise at 10 mL/min using a liquid delivery pump, but the solid polymer did not precipitate, and a mixed liquid containing the polymer solution and the poor solvent was obtained. Next, 300 g of normal heptane as a hydrocarbon dispersion medium was weighed into a 2000 mL separable flask equipped with a stirrer having four inclined paddle blades with a diameter of 5.0 cm, a reflux condenser, and a nitrogen tube, and 0.3 g of a maleic anhydride-modified ethylene-propylene copolymer (trade name: Hi-WAX 1105A, manufactured by Mitsui Chemicals, Inc.) as a polymeric dispersant and 0.3 g of sucrose stearic acid ester (trade name: Ryoto Sugar Ester S-370, HLB value 3, manufactured by Mitsubishi Chemical Foods Corporation) as a surfactant were added, and the mixture was heated and dissolved while stirring at 500 rpm, and then allowed to cool to 70°C. Thereafter, the stirring speed was changed to 1000 rpm, and the above-described mixed liquid (140 g) was added using a pressure filter. After holding at 70°C for 30 minutes, the mixed liquid was heated using an oil bath at 125°C to distill off the solvent, thereby obtaining an aggregate of the polymer. The obtained aggregate was irregularly shaped and could not be classified because its shape was too large.

**[Table 1]**

| | Degree of neutralization of polymer [mol%] | Polymer dispersion liquid (mixed liquid in Comparative Example 3) | | | | Volume ratio of polymer dispersion liquid/dispersion medium | Shape of polymer powder | Median particle diameter of polymer powder [µm] |
|---|---|---|---|---|---|---|---|---|
| | | Polymer concentration [mass%] | Poor solvent concentration [mass%] | Viscosity [mPa·s] | Transmittance [%] | | | |
| Example 1 | 70 | 3 | 35 | 1,810 | 0.63 | 0.50 | True spherical particles | 284 |
| Example 2 | 70 | 3 | 49 | 1,150 | 0.70 | 0.52 | True spherical particles | 275 |
| Example 3 | 70 | 3 | 35 | 1,200 | 0.66 | 0.50 | True spherical particles | 267 |
| Example 4 | 70 | 3 | 40 | 1,110 | 0.10 | 0.51 | True spherical particles | 631 |
| Example 5 | 70 | 2 | 40 | 70 | 0.40 | 0.51 | True spherical particles | 270 |
| Example 6 | 40 | 3 | 40 | 4,360 | 0.51 | 0.51 | True spherical particles | 607 |
| Example 7 | 100 | 3 | 21 | 730 | 0.29 | 0.49 | Substantially true spherical particles | 545 |
| Example 8 | 70 | 3 | 35 | 1,810 | 0.63 | 0.15 | True spherical particles | 165 |
| Example 9 | 70 | 3 | 35 | 1,810 | 0.63 | 0.30 | True spherical particles | 273 |
| Comparative Example 1 | 70 | 3 | 0 | 27,800 | 4.40 | 0.46 | Ellipsoidal particles | - |
| Comparative Example 2 | 70 | 3 | 30 | 4,020 | 7.82 | 0.50 | Irregularly shaped aggregates | - |
| Comparative Example 3 | 70 | 9.5 | 5 | 183,400 | 0.90 | 0.33 | Irregularly shaped aggregates | - |

In Examples 1 to 9, since the solid polymer was precipitated by bringing the poor solvent into contact with the polymer solution so that the transmittance became 0.85% or less, a polymer powder that became true spherical or substantially true spherical in the dispersion medium of the reverse-phase suspension system could be obtained. On the other hand, in Comparative Example 1, since the polymer solution was not mixed with the poor solvent, the solid polymer did not precipitate, and a true spherical polymer powder could not be obtained in the dispersion medium of the reverse-phase suspension system. In Comparative Examples 2 and 3, the polymer barely precipitated upon mixing of the polymer solution and the poor solvent, and the transmittance of the polymer dispersion liquid exceeded 0.85%, and therefore a polymer powder that became true spherical or substantially true spherical in the dispersion medium of the reverse-phase suspension system could not be obtained.

### Reference Signs List

1: water-absorbent resin particles, 10: absorbent body, 20a, 20b: core wrap sheets, 30: liquid permeable sheet, 40: liquid impermeable sheet, 50: water-absorbent sheet, 100: absorbent article.

## Claims

1. A production method for a polymer dispersion liquid, the production method comprising:
a step of preparing a polymer solution comprising a polymer in which a crosslinked structure of a water-absorbent resin has been cleaved; and
a step of bringing a poor solvent for the polymer into contact with the polymer solution to precipitate a solid polymer, thereby obtaining a polymer dispersion liquid in which the solid polymer is dispersed,
wherein a transmittance of the polymer dispersion liquid at a wavelength of 425 nm and an optical path length of 1 cm is 0.85% or less.

2. The production method according to claim 1,
wherein the viscosity of the polymer dispersion liquid at 25°C is 5000 mPa·s or less.

3. The production method according to claim 1,
wherein the poor solvent comprises at least one selected from the group consisting of alcohols having 1 to 10 carbon atoms, phenols, and ketones.

4. The production method according to claim 1,
wherein the degree of neutralization of the polymer is 40 mol% or more.

5. A production method for water-absorbent resin particles, the production method comprising a step of crosslinking the solid polymer in the polymer dispersion liquid obtained by the production method according to any one of claims 1 to 4 to obtain a water-absorbent resin.
